(19) Europäisches Patentamt / European Patent Office / Office européen des brevets

(11) **EP 2 584 966 B1**

(12) **EUROPEAN PATENT SPECIFICATION**

(45) Date of publication and mention
of the grant of the patent:
**26.05.2021 Bulletin 2021/21**

(21) Application number: **11797728.0**

(22) Date of filing: **23.06.2011**

(51) Int Cl.:
*A61B 5/083* (2006.01)   *A61B 5/0205* (2006.01)
*A61B 5/113* (2006.01)   *A61B 5/1455* (2006.01)
*A61B 5/00* (2006.01)

(86) International application number:
**PCT/IL2011/000500**

(87) International publication number:
**WO 2011/161680 (29.12.2011 Gazette 2011/52)**

(54) **METHOD AND SYSTEM FOR SLEEP DISTURBANCE ANALYSIS**

VERFAHREN UND SYSTEM ZUR ANALYSE VON SCHLAFSTÖRUNGEN

MÉTHODE ET SYSTÈME D'ANALYSE DES TROUBLES DU SOMMEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2010 US 357558 P**

(43) Date of publication of application:
**01.05.2013 Bulletin 2013/18**

(73) Proprietor: **Oridion Medical 1987 Ltd.
91450 Jerusalem (IL)**

(72) Inventors:
• **COLMAN, Joshua Lewis
97430 Jerusalem (IL)**
• **LAIN, David
Easton, Maryland 21601 (US)**
• **RONEN, Michal
60948 Givat-Brenner (IL)**

(74) Representative: **Straus, Alexander et al
2K Patent- und Rechtsanwälte - München
Keltenring 9
82041 Oberhaching (DE)**

(56) References cited:
**WO-A2-2008/029399    WO-A2-2009/063446
US-A1- 2006 020 178    US-A1- 2007 191 697
US-A1- 2010 063 366**

**Description**

**FIELD OF THE INVENTION**

The invention relates to a method and system for sleep disturbance analysis.

**BACKGROUND OF THE INVENTION**

A significant number of individuals are prone to brief interruptions (apneas) of breathing during sleep due to a common sleep disorder known as obstructive sleep apnea (OSA). OSA is believed to affect approximately 18 million people in the US and has been linked to an increased risk of heart disease, high blood pressure, stroke, and impotency. Spurred by an increase in the OSA diagnosis rate, the American Academy of Sleep Medicine (AASM) estimates that "the number of patients seeking treatment will grow at a calculated annual growth rate (CAGR) of 16% from 2005-2010."

OSA is a common disease in the United States. The prevalence of OSA, as indicated by data from the Wisconsin Cohort Study (adults aged 30-60 years), is 9-24% for men and 4-9% for women. The estimated prevalence of OSAHS (obstructive sleep apnea-hypopnea syndrome) is 2% in women and 4% in men.

The exact cause of OSA remains unclear. The site of obstruction in most patients is the soft palate, extending to the region at the base of the tongue. There are no rigid structures, such as cartilage or bone, in this area to hold the airway open. During the day, muscles in the region keep the passage wide open. But as a person with OSA falls asleep, these muscles relax to a point where the airway collapses and becomes obstructed. When the airway closes, breathing stops and the sleeper awakens to open the airway. The arousal from sleep usually lasts only a few seconds, but brief arousals disrupt continuous sleep and prevent the person from reaching the deep stages of slumber, such as rapid eye movement (REM) sleep, which the body needs in order to rest and replenish its strength.

Many types of abnormal breathing during sleep have been described as apneas. Partial airway obstruction can lead to a reduction in tidal volume, referred to as progressive increases in respiratory effort, reflecting increasing upper airway resistance. Some patients have periods of hypoventilation during sleep, most commonly seen in REM, and not always associated with apneic events.

Another type of breathing abnormality consists of those apneic events that are not associated with inspiratory effort, indicating reduced central respiratory drive, referred to as central apneas.

As different types of disordered breathing events during sleep have been described, it has been recognized that signs and symptoms could be used to describe several syndromes.

Guilleminault introduced the term obstructive sleep apnea syndrome (OSAS) with its central feature of daytime hyper-somnolence and polysomnographically proven obstructive apneas. Hypopneas were first described as events of shallow breathing causing oxygen desaturation. In 1988, cases with Hypopneas and no or few apneas were described, with clinical symptoms similar to OSAS, and introduced the sleep hypopnea syndrome. Subsequently the OSAS began to be referred to as the obstructive sleep apnea-hypopnea syndrome (OSAHS).

The initial description of OSAS by Guilleminault included a criterion of a minimum duration of 10 seconds for an apnea to be scored. Based on a study of healthy subjects it was also suggested that more than 30 apneas per night should be considered abnormal. This was later standardized as the apnea index, which reflects the number of apneas per hour of sleep.

Since the initial descriptions of these different types of abnormal breathing events and their related syndromes, technology has changed and original definitions have been modified.

Today, the diagnosis of sleep disorders commonly includes both a patient clinical assessment and an overnight recording session, where several patient parameters are recorded. The recorded data is later analyzed by an expert and/or dedicated software program to detect and provide a score to the level of sleep disorder.

Two of the basic patient parameters used to determine the severity of the sleep disorder is the apnea-hypopnea index (AHI) and the oxygen desaturation index, both indicative of a fall in respiratory effort or drive. Though an apnea event is the result of a total cessation of breathing, for a hypopnea event, it is sufficient that the patient breath flow drops measurably (for example, by more than 50%). It is generally accepted that detection of hypopnic events even without total apnea is sufficient for OSA diagnosis. Though the hypopnea index and the many correlating arousals are a clear indicator of the disorder, it is believed that the resulting periods of hypoxia are the main reason for the increased patient health risks.

During a sleep study, many parameters relating to the patient's sleep are recorded and later analyzed, either by an expert and/or an appropriate software package. All the hypopnea and apnea events during the night are counted, and indexes for each separately and also together are calculated, based on the average number of events per hour. It is accepted that the higher the index calculated is, the severity of OSA is greater, where three levels are often defined: mild, moderate and severe. The medical decision or diagnosis does not rely only on this value, but it is considered as one of the main parameters for decision making.

Though as mentioned above, it is felt by many experts that it is the resulting periods of hypoxia that are the main reason for the increased patient health risks, an oxygen desaturation index is not always calculated by itself, but is taken into consideration via the hypopnea index, which, as defined, requires a subsequent desaturation event in order for it to be counted (though, according to the AASM definition, an arousal could replace the need for the desaturation).

Many studies have been made over the years to correlate sleep scores using the AHI (apnea hypopnea index) with OSA severity, patient populations, outcomes like hypertension etc., with often conflicting results.

Although sleep medicine, its understanding and knowledge have increased exponentially over the past 20 years, many inherent drawbacks in the technical definition and method of measurement of the AHI exist. The mere fact that the AHI is created by calculating the number of events per hour, where all events are weighted equally, is believed to be a major drawback. One event may be far stronger than another, both per patient and relative to different patients, and the equal treatment these events receive may cause numerous inherent errors in the diagnosis of sleep disorders.

Further, the AHI index is based mainly upon measurements of the patient flow, where the measure of flow is an indirect manner of measuring tidal volumes. Where it is the tidal volume, which truly indicates the effectiveness of the ventilation and oxygenation, and as explained, will dictate the sleep quality. The correlation between volume and flow is often poor, and is further dependent on the duration of the breath and how it changes over the breath cycle. Still further, when patient breath flow is measured, what is normally being measured is relative flow without any absolute measurements. This, since it may be virtually impossible to measure total flow without closing off the entire oral/nasal cavities to permit the total breath to pass via the flow sensor. In reality, one samples only a part of the air flowing from the nasal and/or oral nasal cavity, in order to provide a value that is supposed to be proportional to the entire flow and that, proportional to tidal volume. However, as explained, one can change a level of flow without changing volume, by changing the pattern or length of the breath, mouth aperture or mode of breathing, i.e. nasal, oral or oral nasal.

Currently, in many sleep test settings, assessment of sleep quality, as related to effective breathing, is carried out by calculating an Apnea Index (AI) and a Hypopnea Index (HI), and/or their sum, the Apnea-Hypopnea Index (AHI). These indices are based on counting events during sleep period and averaging them over time (e.g., number of events per hour). The calculation of events is based on parameters collected using flow sensors (pressure and or thermocouple sensors), oximeter and or EEG. The events are in general a change (usually, a decrease) in the level of the parameter from a base-line for at least a predefined amount of time, followed by a change in the opposite direction (usually, an increase). For example an Apnea event as defined by the American Association of Sleep Medicine may be a decrease to 90% in the pressure level, as compared to its base-line, that lasted 10 seconds as measured by a thermistor. The indices are quantitative, as all events are counted equal, disregarding their severity and their duration (e.g., a de-saturation event that lasts 60 seconds, with a drop of $SpO_2$ from 95% to 85%, has the same weight as a de-saturation event that lasts 6 seconds with a drop of $SpO_2$ from 100% to 95%).

The common diagnostic criteria of a sleep disorder is that the individual fulfills criterion A or B, plus criterion C, as presented below, to be regarded as suffering from a sleep disorder:

A. Excessive daytime sleepiness that is not better explained by other factors;
B. Two or more of the following that are not better explained by other factors:

- choking or gasping during sleep,
- recurrent awakenings from sleep,
- unrefreshing sleep,
- daytime fatigue,
- impaired concentration;

C. Overnight monitoring demonstrates five or more obstructed breathing events per hour during sleep. These events may include any combination of obstructive apneas/hypopneas or respiratory effort related arousals, as defined below.

The American Academy of Sleep Medicine (AASM) recommends that an apnea event be detected using an oral-nasal thermal sensor, which measures the respiratory gas temperature that is proportional to the flow passing across it, while for detection of a hypopnea event they recommend the use of a pressure transducer where changes in pressure are proportional to the flow. Though the AASM recommend the use of the thermal sensor for apnea, they propose, as an alternative, the use of a pressure transducer. The pressure transducer method uses a cannula interfaced to the patient to capture the breath sample, where the pressure created from the breath flowing into the cannula is sensed by a pressure sensor positioned at the end of the cannula. Though it is not mentioned in the manual, it is normal to find that the cannula is an oral-nasal cannula, in order to sample both nasal and oral breathing. However, many sleep labs prefer to use only a nasal cannula for that purpose.

Though there are many definitions of a hypopnea event, the following is one of the most accepted definitions:

A) <u>Medically</u>: Hypopnea is a medical term for a disorder which involves episodes of overly shallow breathing and/or an abnormally low respiratory rate. This differs from apnea in that there still remains some flow of air. Hypopnea events may happen while asleep or while awake.

B) <u>Technically</u>: A reduction of airflow lasting 10 or more seconds measured by a 30 percent reduction in airflow, associated with a drop in oxygen level by 4 percent, or a 50% reduction in airflow accompanied by either a 3% drop in oxygen level or an arousal. Hypopneas are often said to have the same clinical significance as an apnea.

An apnea event (as defined for sleep studies), unlike a hypopnea event, is sometimes defined as a 90% detected reduction in flow, as measured by either a pressure or a thermal sensor, but does not require a corresponding desaturation event. Again, a 10 second period is usually required in order for it to be considered and counted as an event.

Further drawbacks are laid out below:

**Drawbacks in the use of flow as the parameter to measure shallow breathing:**

As defined above, a hypopnic event is often medically defined as involving episodes of overly shallow breathing and/or an abnormally low respiratory rate. These overly shallow breaths may be caused by partial obstruction of the airway, or may be of a central nature, where the source is metabolic and/or neuromuscular. This reduction in strength, effort and/or drive will normally create a drop in tidal volume of the breath and minute ventilation, and a consequent reduction of effective gas transfer (Oxygen intake and $CO_2$ removal). Even in normal breathing, some 20% of the breath will not take part in gas transfer; this percentage is the result of dead space, which is the volume from the mouth down to the bronchiole tubes where the breath does not make contact with the Alveoli (where breath transfer takes place). This volume is approximately 150ml in adults, relative to a normal tidal volume of some 500ml. Hence the more one breaths shallowly, the percentage of breath taking part in gas transfer will drop strongly, this, since the dead space volume will remain constant but the tidal volume will fall.

It would infer from the above, that optimally the actual changes in tidal volume or minute ventilation during sleep should be measured. Instrumentation for these purposes may be spirometry or pneumotachography, respectively, which dictates measuring volumes of the entire breath. This, however, is not very practical during sleep. Spirometry, for example, is accomplished normally in controlled measuring procedures with a cooperative patient.

The measure of flow is hence an *indirect* manner of measuring tidal volumes, where the correlation between volume and flow will be dependent on the duration of the breath and how it changes over the breath cycle.

Further, when patient breath flow is measured, what is normally being measured is relative flow without any absolute measurements. This, since it may be virtually impossible to measure total flow without closing off the entire oral/nasal cavities to permit the total breath to pass via the flow sensor. In reality, one samples only a part of the air flowing from the nasal and/or oral nasal cavity, in order to provide a value that is supposed to be proportional to the entire flow. However, as explained, one can change a level of flow without changing volume, by changing the pattern and length of the breath.

**Drawbacks in the method of measurement:**

As mentioned, two methods for measurement are commonly recommended by the experts and AASM: Thermal or pressure sensors. The pressure sensor method has two main drawbacks:

***1. Issues relating to the sampling means and interface:***

Optimally, a patient should be sampled from both his nasal and oral cavities, this, since a patient can have a mixture of nasal, oral and oral nasal breathing (though nasal breathing is most prominent in patients with OAS, who tend to open their mouths during sleep).

Sampling from both nostrils and mouth dictates a more complicated interface design, often considered cumbersome during sleep. Many marketed products are nasal alone for this reason. Further, even if an oral-nasal design is used and tolerated, the oral sampling prong must cover a substantial part of the mouth in order to capture flow and changes in order to be useful.

Further, the measured flow level will be dependent on where the patient is breathing from; if the patient changes his breathing characteristic, i.e. from pure nasal to oral-nasal etc., a change of flow will be sensed, though in reality the total flow may remain the same.

Still further, the level of flow measured will depend on the extent of the opening of the mouth. When wider open, the flow captured will naturally fall. And still further, a partially blocked nose or intermittently blocked nostril will also change the measured flow shape. All the above changes could be detected as an erroneous hypopnea, though none were the result of a partial blockage or shallow breath. Further, as mentioned, the flow levels measured are relative, with no absolute value, and will differ from patient to patient based, for instance, on the size of the patient' nostrils relative to the sampling cannula.

## 2. *Issues related to correlating pressure measurements to actual flow:*

Pressure, as mentioned, is an *indirect* measure of flow (proportional to the square root). Different sensors and systems use different methods to measure and depict the flow.

Still further, many systems prefer to use an AC coupled measuring system, which is sensitive only to changes in pressure and not constant flow. This, since the cannula may be placed under a CPAP (continuous positive airway pressure) mask, where the high pressures would interfere with the measurements if DC coupling would be used. This AC coupling acts like a filter and hence also changes the shape of the real flow changes.

## Drawbacks in the technical definition of Hypopnea when measured with flow:

As mentioned above, a technical definition of hypopnea has been provided which is relevant to when measuring flow with a pressure transducer, i.e. a reduction of airflow lasting 10 or more seconds measured by a 30 percent reduction in airflow, associated with a drop in oxygen level by 4 percent or a 50% reduction in airflow accompanied by either a 3% drop in oxygen level or an arousal. Hypopneas are usually related to as having the same clinical significance as an apnea.

In order to define a reduction in flow of 30 or 50%, one must define a baseline from where it has fallen. This baseline must be updated continuously, since not only fast changes occur, but also gradual changes. Then the question is asked, how to calculate this baseline? How often is it updated? After a fall, how does one reset it? From which point is it reset? How does one take into consideration that the change is possible caused by a change in flow pattern or breath period? From where does one count the 10 seconds?

## Drawbacks based on the fact that all events are defined equally:

All events are calculated in the index as equal, though one may ask, is the effect on a patient is the same? Is an event that lasted 10 seconds the same as one that lasted 40 seconds? Is a desaturation event that had a 3% drop similar to one where it dropped by 13%? If an $SpO_2$ baseline was 99% or 89%? If the flow fell by 30% or fell by 80%? Is there importance to recurring patterns? And so on.

Hence, there is a long-felt need for an improved standard method with more absolute means of scoring apnea/hypopnea events.

## Prior Art:

WO 2009063446 A2 discloses a method of generating a pulmonary index value of a patient, which includes receiving two or more measured patient parameters, wherein at least one of the measured parameters originates from a pulmonary sensor; and computing the pulmonary index value based on the two or more measured patient parameters.

WO 2008029399 A2 discloses a method for diagnosis including receiving a signal associated with blood oxygen saturation of a patient during sleep. The signal is filtered to eliminate signal components at frequencies equal to and greater than a respiratory frequency of the patient. The filtered signal is processed to detect a pattern corresponding to multiple cycles of periodic breathing.

US 2007191697 A1 discloses a system and method for analyzing data. As an example the method comprises receiving data corresponding to at least one time series, and computing a plurality of sequential instability index values of the data. Further it comprises a source of data indicative of at least one time series of data, and a processor that is adapted to compute at least one of a plurality of sequential instability index values of the data.

## SUMMARY OF THE INVENTION

There is provided, in accordance with an embodiment, a method for automatic sleep test analysis, the method comprising: continuously monitoring, using a pulse oximeter, oxygen saturation values of a patient; continuously monitoring, using a capnograph, exhaled carbon dioxide ($CO_2$) values of the patient; and computing a continuous integrated sleep score based on a sequential analysis of the oxygen saturation values and the exhaled $CO_2$ values, wherein the continuous integrated sleep score is indicative of the patient's breathing-related sleep quality during at least a portion of the sleep test. The method comprises detecting multiple events indicative of ineffective breathing exhibited in one or more of the oxygen saturation values and the CO2 values; and computing a weighting function for the detected events, wherein each event is assigned a suitable weight. The suitable weight for each event is based on applying an integrative function to the oxygen saturation values and CO2 values over a duration of each event, wherein the integrative function comprises the following parameters: the oxygen saturation values and CO2 values over the duration of the event; baseline oxygen saturation and CO2 values measured prior to the event; and the duration of the event.

In one embodiment, the integrative function further comprises at least one parameter selected from the group consisting of: a difference between minimum oxygen saturation and CO2 values over the duration of the event and the baseline oxygen saturation and CO2 values; a maximal difference between minimum oxygen saturation and CO2 values over the duration of the event and the baseline oxygen saturation and CO2 values; and minimal oxygen saturation and CO2 values over the duration of the event.

There is further provided, in accordance with an embodiment, a system for automatic sleep test analysis, the system comprising: a pulse oximeter for continuously monitoring oxygen saturation values of a patient; a capnograph for continuously monitoring exhaled carbon dioxide ($CO_2$) values of the patient; and a computing unit configured to compute a continuous integrated sleep score based on a sequential analysis of the oxygen saturation values and the exhaled $CO_2$ values, wherein the continuous integrated sleep score is indicative of the patient's breathing-related sleep quality during at least a portion of the sleep test. The system comprises detecting multiple events indicative of ineffective breathing exhibited in one or more of the oxygen saturation values and the CO2 values; and computing a weighting function for the detected events, wherein each event is assigned a suitable weight. The suitable weight for each event is based on applying an integrative function to the oxygen saturation values and CO2 values over a duration of each event, wherein the integrative function comprises the following parameters: the oxygen saturation values and CO2 values over the duration of the event; baseline oxygen saturation and CO2 values measured prior to the event; and the duration of the event.

In one embodiment, the integrative function further comprises at least one parameter selected from the group consisting of: a difference between minimum oxygen saturation and CO2 values over the duration of the event and the baseline oxygen saturation and CO2 values; a maximal difference between minimum oxygen saturation and CO2 values over the duration of the event and the baseline oxygen saturation and CO2 values; and minimal oxygen saturation and CO2 values over the duration of the event.

In some embodiments, the sequential analysis comprises applying to the oxygen saturation values and the exhaled $CO_2$ values an analysis method selected from the group consisting of: a linear regression model, a non-linear regression model, fuzzy logic, a Bayesian network, a decision tree, a neural network, a radial base function and an expert system.

In some embodiments, the method further comprises computing, from the continuous integrated sleep score, an integrated sleep score for each of a plurality of time windows in the sleep test.

In some embodiments, the time windows are moving time windows.

In some embodiments, the moving time windows are each of a static length.

In some embodiments, the method further comprises dynamically changing a length of at least some of the moving time windows.

In some embodiments, the method further comprises monitoring one or more additional parameters selected from the group consisting of: heart rate, encephalogram (EEG), breath flow and chest movement, wherein the computing of the continuous integrated sleep score is further based on one or more of the additional parameters.

In some embodiments, the method further comprises summarizing at least a portion of the continuous integrated sleep score, to generate a summarized sleep quality score.

In some embodiments, the computing unit is further configured to: detect multiple events indicative of ineffective breathing exhibited in one or more of the oxygen saturation values and the $CO_2$ values; and compute a weighting function for the detected events, wherein each event is assigned a suitable weight.

In some embodiments, the computing unit is further configured to compute, from the continuous integrated sleep score, an integrated sleep score for each of a plurality of time windows in the sleep test.

In some embodiments, the computing unit is further configured to dynamically change a length of at least some of the moving time windows.

In some embodiments, the system further comprises at least one apparatus selected from the group consisting of: a heart rate monitor; an electroencephalogram (EEG) monitor; a breath flow sensor; and a chest movement sensor, wherein the computing is further based on data received by the computing unit from one or more of the apparatuses.

In some embodiments, the computing unit is further configured to summarize at least a portion of the continuous integrated sleep score, to generate a summarized sleep quality score.

## BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. The figures are listed below.

EP 2 584 966 B1

Fig. 1 shows a semi-pictorial illustration of a patient connected to sleep test equipment;
Fig. 2 shows a flow chart of a method for automatic sleep test analysis;
Fig. 3 shows an exemplary flowchart in which input parameters from a capnograph, a pulse oximeter and a flow meter are analyzed over a time window and applied with a transformation function;
Fig. 4 shows a flow chart in which, after calculating a continuum or a set of single, integrated sleep scores, a sleep quality score (SQS) is calculated;
Fig. 5 shows a transformation function for $EtCO_2$ with exemplary values;
Fig. 6 shows values of a continuous integrated sleep score (CISS) for $EtCO_2$ over time;
Fig. 7 shows the use of linear combination of $EtCO_2$ and $SpO_2$ to generate the CISS;
Fig. 8 shows $CO_2$ concentrations (waveforms) with continuous scoring between 0 and 10;
Fig. 9 shows $SpO_2$ values and a calculated score, 0 to 100; and
Fig. 10 shows an integrated score, 0 to 100.

**DETAILED DESCRIPTION**

An aspect of some embodiment relates to the usage of a capnograph in sleep testing, instead of or in conjunction with a flow meter. Capnography is often said to be one of the most sensitive monitors for tracking patient ventilation available today. A flow meter can often provide an erroneously higher score because changes in patient breathing patterns can be misinterpreted for a hypopnic event.

Although a capnograph may not be primarily used to detect shallow breathing, it does provide absolute measurements, not relative, which are directly linked with breathing performance and ventilation.

A capnograph may still be used in conjunction with a pulse oximeter, since the consequent hypoxia events indicated by desaturation and level of $SpO_2$ are linked more with the risks, than the shallow breathing, and for which reason a hypopnea event is defined as one which occurs together with a desaturation event.

A capnograph operating together with a pulse oxymeter may provide a more reliable method for not only detection of sleep disturbance but to provide a better means for evaluating the severity. Further, as mentioned, a shallow breath will likely have a corresponding effect on the $CO_2$ level, when falling towards dead space breathing, hence a falling $CO_2$ level is more indicative of a hypopnic event that is harmful to the patient than flow alone, and its absolute value corresponds more than the indirect measure of flow.

Further, an oral-nasal cannula for measuring $CO_2$ is not normally affected by changing breathing patterns, i.e. nasal to oral and vice versa, neither is it dependent on the opening of the mouth. Additionally, not only is the $CO_2$ waveform amplitude indicative of ventilation performance, but also are the inherent shapes of a single waveform and the patterns of groups of waveform. Using all these attributes together may provide a more robust means for analyzing the quality of sleep.

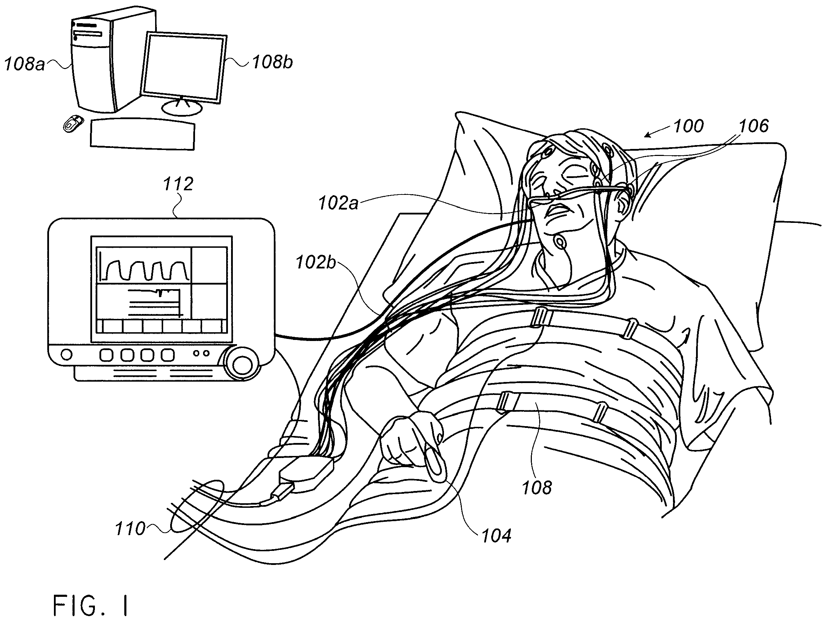

Reference is now made to Fig. 1, which shows a semi-pictorial illustration of a patient **100** connected to sleep test equipment. An oral-nasal cannula **102a** may be positioned on the patient's face, for collecting exhaled oral and nasal breath. The exhaled breath is drawn through a $CO_2$ tube **102b** towards a capnograph **112** which may be positioned next to the patient's bed. A pulse oximeter **104** may be positioned on the patient's finger, for measuring oxygen saturation values.

Optionally, various other sensors may connected to or associated with the patient. For example, one or more electrodes **106** may be attached to the patient's face and/or scalp, for measuring eye movement, brain activity and/or the like. One or more elastic belts **108** may be worn by the subject, measuring his or hers chest movement, which is indicative of breath.

Cables **110** of the different sensors may extend up to a monitoring room of the sleep lab, if the sleep test is performed in a lab. There, the cables may be connected to a computing unit **108a** which receives and analyzes the data. A monitor **108b** may be connected to computing unit **108**, for the purpose of displaying one or more of the sleep-related scores in real time, as they are being generated. Optionally, a printer (not shown) may also be connected to computing unit **108a**, for printing a summary score at the end of the sleep test, and/or the individual scores that were generated throughout the test period. The printed scores may be laid out graphically and/or numerically. Optionally, a graphical and/or numerical report is generated as a digital file by computing unit **108** without printing.

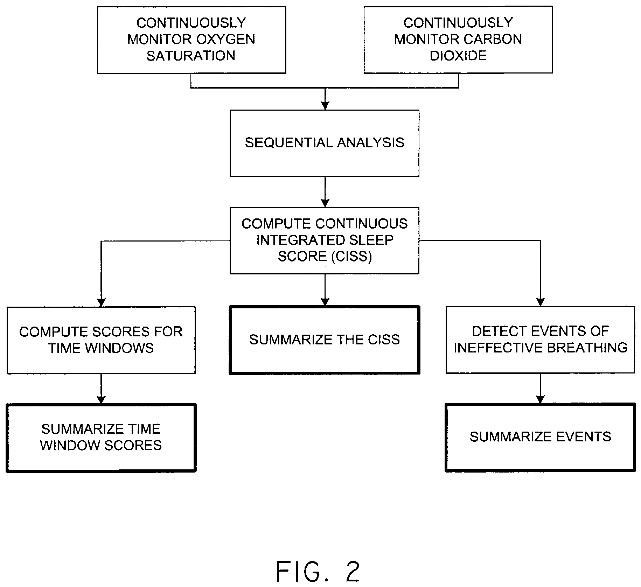

In an embodiment, there is provided a method for automatic sleep test analysis, in which oxygen saturation is monitored by pulse oximeter **104** of Fig. 1, $CO_2$ is monitored by capnograph **112** of Fig. 1, and one or more sleep-related scores are computed by computing unit **108** of Fig. 1. The method is shown schematically in Fig. 2.

The method includes receiving two or more measured patient parameters at a given time window during sleep, wherein the at least two measured parameters relate to the breathing effectiveness and originate from a Capnograph and a Pulse Oximeter; and computing a single, integrated sleep score (ISS) based on the two or more measured patient parameters during that time window indicative of the sleep quality and effectiveness. Accordingly, multiple integrated sleep scores are being computed in each sleep test.

7

Secondly and optionally, for a period of time greater than the said time window, a continuum or set of scores may be computed, generating a continuous integrated sleep score (CISS).

Thirdly and also optionally, a summary of the continuous integrated sleep score or of the multiple integrated sleep scores of a patient, which includes receiving and using the CISS or ISSs for calculating a summary score to be referred as the Sleep Quality Score (SQS). The SQS may be computed for the whole duration of sleep test or for sub-period or sub-periods of the sleep-test (for example for periods with a specific posture or periods with a specific sleep-stage). Additional parameters may be received and used for the definition of the sub-period ranges. The method may further include providing a medical recommendation.

Advantageously, the computed CISS and/or SQS provide a measure that is based on quality rather than on quantity alone.

According to some embodiments, the single, integrated scores may be calculated by various means, such as, for example, by use of mathematical equations, algorithms, formulas, and the like that may take into consideration one or more of the values or derivatives of the values of the parameters.

According to some embodiments, computing the single, integrated scores may include applying a mathematical model reflecting medical expert considerations, literature, clinical data, medical experience or any combination thereof that indicate the quality of the sleep as reflected by the measured parameters. Computing the single, integrated scores may include applying a linear regression model, a non-linear regression model, a fuzzy logic, a Bayesian network, a decision tree, a neural network, a radial base function, an expert system, or any combination thereof.

According to further embodiments, the at least two measured parameters may include a $CO_2$ related parameter and an $O_2$ related parameter. In addition, the heart rate, an encephalogram (EEG), breath flow (using a pressure transducer and/or a thermocouple), chest movement, or any combination thereof may optionally be used.

The $O_2$ related parameter may include $SpO_2$, change in $SpO_2$, slope of change in $SpO_2$ their variability's or any combination thereof.

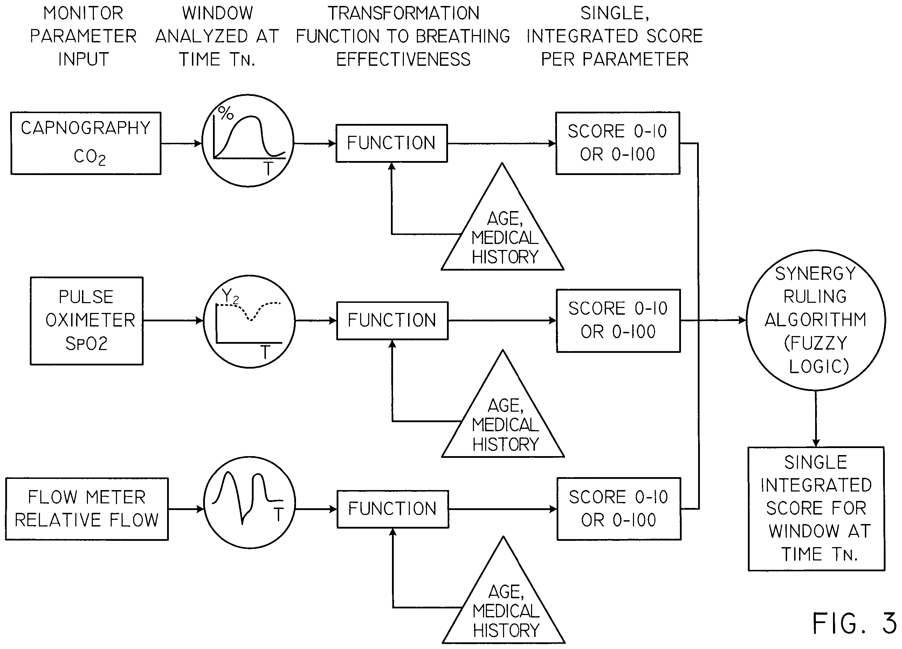

Fig. 3 shows an exemplary flowchart, in which input parameters from a capnograph, a pulse oximeter and a flow meter are analyzed over a time window and a transformation function is applied to each one of them. Then, a single integrated score per parameter is calculated, followed by a synergy ruling algorithm which is used to generate a single, integrated score for that time window, based on all per-parameter scores.

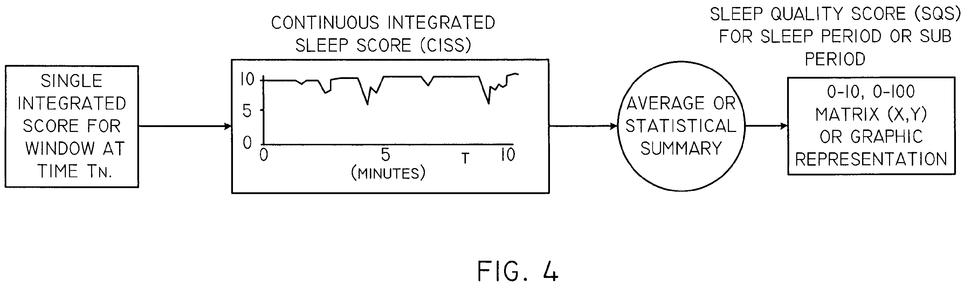

Fig. 4 shows a flow chart in which, according to an embodiment, after calculating a continuum or set of single, integrated sleep scores that generates a continuous integrated sleep score (CISS), the sleep quality score (SQS) is calculated based on a mathematical assessment of the CISS for the period of interest and represented as either a single score, or a set of scores, or matrix.

According to additional embodiments, the method of calculating the single integrated sleep score and/or SQS of a subject may further include receiving one or more subject characteristics; and computing the single integrated sleep score and/or SQS based on the two or more measured patient parameters and on one or more patient characteristics. The one or more patient characteristics may include age, weight, gender, medical condition, medication, medical history or any combination thereof. The medical history may include smoking, heart disease, lung disease or any combination thereof.

According to further embodiments, the single integrated sleep score may be on a simple scale such as the range of 1 to 100 or 1 to 10. It is therefore a unit-less score which is only indicative of the magnitude of the sleep quality as a continuous value during sleep test. Where either the lowest value indicates a high quality of sleep or the highest value indicates the high quality of sleep.

According to additional embodiments, the method of generating the single integrated sleep score and or SQS of a patient may further include computing a reliability index of the SQS.

According to further embodiments, the SQS may be calculated by various means, such as, for example by use of mathematical equations, algorithms, formulas, and the like that may take into consideration one or more of the values or derivatives of the values of CISS, such as average, median, integration, variance and the like or combination thereof.

According to some embodiments, computing the SQS may include applying a mathematical model reflecting the overall sleep quality at the period of sleep or at parts of the period of sleep.

According to a further aspect there is provided a method for calculating an overall score, the sleep quality score which is based upon the single quality sleep scores generated over a trial period. The SQS can also be calculated for a sub-period of the trial for example per hour or period of sleep mode or patient position. The SQS may be calculated using an average or any other standard statistical means for depicting and representing a set of scores over a period of time. The sleep quality score is hence a value indicative of the overall sleep quality for the period defined. The sleep disorder measure may be represented by a single value or by a set of values or even graphic representation. The set of values may include one value generated by the statistical evaluation of the single integrated scores, while a second related to number or rate of events. While a third may relate to distribution, standard distribution or any other statistical assessment of the CISS.

According to yet additional embodiments, the method may further include a graphic display of the CISS. The score can be calculated in real time, off-line, at the apparatus as is, or combined to other apparatus tool such as the polysomnograph (PSG). Optionally, the information from the capnograph, pulse oximeter and optionally flow meter is transmitted via a communication port directly or wirelessly to a PSG or other sleep analysis tool.

There may be a need to analyze the various patient related parameters that are collected during sleep, and provide the sleep test analyzers and technicians with a more concise, comprehensive, intuitive, clear and useful information about the patient's sleep quality as a result of the breathing effectiveness.

The computed CISS and SQS provide a measure that is based on quality rather than on quantity alone.

The CISS and SQS may be calculated on real-time or off-line using the parameters data. The single integrated sleep score from which the CISS and SQS are generated, at a given time point reflects the quality of sleep during a previous time window, ranging from few seconds to few minutes. The score is updated at a rate smaller than the minimal time window (moving window). The time window may be an absolute constant (static) or adaptively change (dynamic).

According to some embodiments, the single integrated sleep score computation may be a linear function or a non-linear function or a non-linear transformation of the parameters and or the parameters derivatives indicative of the breathing effectiveness. Non-linear transformations may be applied on each of the parameters separately and later to be integrated into single score or it may be applied on an integrated value of the parameters.

According to some embodiments, the single integrated sleep score computation may be a linear function or a non-linear function or transformation of a parameter in a given time-window taking into account the absolute value of the parameter at any time point in the time-window, the absolute values of the minimum and maximum of the parameter and the difference between them, the rate of change in the parameter values, or any combination thereof.

According to some embodiments, the single integrated sleep score computation may be a linear function or a non-linear function or transformation of a parameter in a given time-window taking into account also an absolute base-line calculated from a different and normally longer time window of the parameter, an absolute base-line value of the parameter whose importance and weighting depends on the subject's characteristics (such as: age, presence of heart-disease, pulmonary disease), an absolute or a relative base-line that depends on other parameters used for the sleep test (such as: sleep stage, time since falling asleep)

According to some embodiments, the single integrated sleep score computation may be a linear function or a non-linear function or transformation of several parameters where a first parameter's value or any of its derivatives (such as: maximum, minimum, differences, rate of change, baseline) may be used in combination with a second parameter's value or its derivatives and hence creating synergy between the input parameters and their integrated effect as related to breathing effectiveness..

According to some embodiments, the single integrated sleep score computation may be event based, where an event of a parameter is detected in a time-window and a weighting function is calculated for each event. The events may include the following: no-breathing for at least Y seconds, a decrease of at least X% of $SpO_2$ values for at least Y seconds, a decrease or an increase of at least X mmHg of $EtCO_2$ values for at least Y seconds, a decrease to at most X mmHg of $EtCO_2$ values for at least Y seconds, an increase to at least X mmHg of $EtCO_2$ values for at least Y seconds, a decrease of successive $EtCO_2$ values with at least Z rate for at least Y seconds, an increase of successive $EtCO_2$ values with at least Z rate for at least Y seconds, a very short breath (spike), decrease in flow (pressure and or thermo-couple) by at least X% for at least Y seconds or any combination thereof.

According to some embodiments, the changes in the parameter values (decrease or increase) are relative to a base line. The base-line may be calculated continuously (i.e. updated every Y seconds, where Y is 1-5 seconds) by averaging (using mean or median or other averaging function) the parameter value in a defined time window. This time-window is selected in a way that the variability of the parameter at that time period is low and no event is included. The variability can be calculated using standard-deviation, range of change, maximum, minimum, variance or the like. For example, base-line for $SpO_2$ may be calculated in a time-window lasting between the last 10-200 seconds, where $SpO_2$ values during that time varies less or equal to $\pm1\%$.

In some embodiments, a flow meter (based on, for example, pressure, thermo-couple and/or the like) may be added to the capnograph and the pulse oximeter. As mentioned, a change in the flow does not necessarily dictate any change in $CO_2$ - such as during a hypopnea event where shallow breathing occurs. Hence, when a reduction in the flow is seen, without any substantial change to the $CO_2$, it would cause a certain drop in the score; however, if the decrease in the flow it is also coupled with a drop in $CO_2$, then the drop in the score may be more significant, since the reduction is now supported by two parameters and not by flow only.

In some embodiments, other one or more parameters commonly monitored in a sleep test using a polysomnograph may be added. For example, readings from a polysomnograph may be indicative on an arousal of the patient. Such arousal, if occurring following or simultaneously with an apnea and/or a hypopnea event, may cause the score to decrease, due to two reasons: Firstly, the arousal is reinforcement of the determination of a apnea/hypopnea event, since it usually occurs when the patient chokes or doesn't breath sufficiently. Secondly, the arousal itself means that the quality of the sleep is low, and, additionally, the occurrence of multiple arousals during the sleep period may consequently cause other problems, such as daytime drowsiness, sleep deprivation, headaches and/or the like.

According to some embodiments, the weighting function calculated **212** for each event may be linear or non-linear function taking into account the absolute value of the parameter at any time point during the event, the absolute values of the minimum and maximum of the parameter and the difference between them, the rate of change in the parameter values, the event's base-line, the difference between the parameters values and the event's base-line, the integral, the difference between the extreme parameter value (e.g. its minimum), the event's base-line, the duration of the event or any combination thereof.

According to some embodiments, the weighting function calculated **212** for each event may be linear or non-linear function taking into account values and or events of other parameter or parameters in a time window larger than the event duration, previous to the event, during the event and or after the event.

According to some embodiments, the single integrated sleep score during an event of a parameter or during events of more than one parameter may be computed as a linear or non-linear function of the event weighting-function value or of the events weighting-functions values. The single integrated sleep score may be a constant value during the period of event or events or it may be a continuous value that changes during the course of the event relative to the extent of the event as defined and inherent to the function used

According to some embodiments, when no event is detected, the single integrated sleep score may be computed based on the parameter's values and on the subject's characteristics (such as: age, presence of heart-disease, pulmonary disease), and or on other parameters used for the sleep test (such as: sleep stage, time since falling asleep). For example, when no events occur, the single integrated sleep score of a healthy subject may be 100 out of 100, if all his parameters are in the normal range. However, if a healthy subject has a low $SpO_2$ (e.g., 90%), but no event is detected his single integrated sleep score may decrease to 80. A subject with a chronic pulmonary disease, with a "normally" low $SpO_2$ levels (e.g., 92% as compare to 98-100% for healthy subjects), may have a single integrated sleep score of close to a 100.

According to further embodiments, the SQS i.e. the summary of the CISS, may be calculated by various means, such as, for example by use of mathematical equations, algorithms, formulas, and the like that may take into consideration one or more of the values or derivatives of the values of the parameters, such as average, median, integration, variance and the like. Hence, depicting the overall sleep quality and breathing effectiveness over the test period.

According to some embodiments, the single, integrated scores may be calculated by various means, such as, for example by use of mathematical equations, algorithms, formulas, and the like that may take into consideration one or more of the values or derivatives of the values of the parameters.

According to some embodiments, computing the single, integrated scores may include applying a mathematical model reflecting medical expert considerations, literature, clinical data, medical experience or any combination thereof that indicate the quality of the sleep as reflected by the measured parameters. Computing the single, integrated scores may include applying a linear regression model, a non-linear regression model, a fuzzy logic, a Bayesian network, a decision tree, a neural network, a radial base function, an expert system, or any combination thereof.

The $CO_2$ related parameter may include a $CO_2$ waveform related parameter, an expired air $CO_2$ concentration, respiratory rate or any combination thereof. The $CO_2$ waveform related parameter may include $EtCO_2$, changes in $EtCO_2$, a slope of the increase in the $CO_2$ concentration, a change in a slope of the increase in the $CO_2$ concentration, time to rise to a predetermined percentage of a maximum value of $CO_2$ concentration, a change in time to rise to a predetermined percentage of a maximum value of $CO_2$ concentration, an angle of rise to a predetermined percentage of a maximum value of $CO_2$ concentration, a change in an angle of rise to a predetermined percentage of a maximum value of $CO_2$ concentration, breath to breath correlation, a change in breath to breath correlation, a $CO_2$ duty cycle, a change in $CO_2$ duty cycle breath duration, change in cycle breath duration or their variability's or any combination thereof.

**Example 1**

An example of functions and generation of the CISS for the *continuous type* of calculation are presented below.

A trapezoidal-shaped membership function, which is a nonlinear function (which is used commonly with Fuzzy-Logic) may be applied on any value of $EtCO_2$ and/or $SpO_2$. The function is designed in a way that input values at the normal range of that parameter will have a score of 1; as the value gets farther from the normal range, the score will decrease.

The general function:

$$f(x, a, b, c, d) = max\left(min\left(\frac{x-a}{b-a}, 1, \frac{d-x}{d-c}\right), 0\right)$$

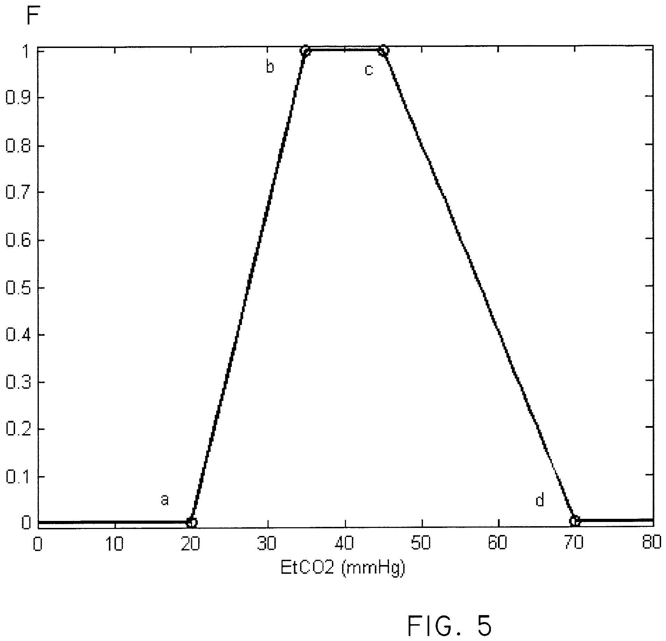

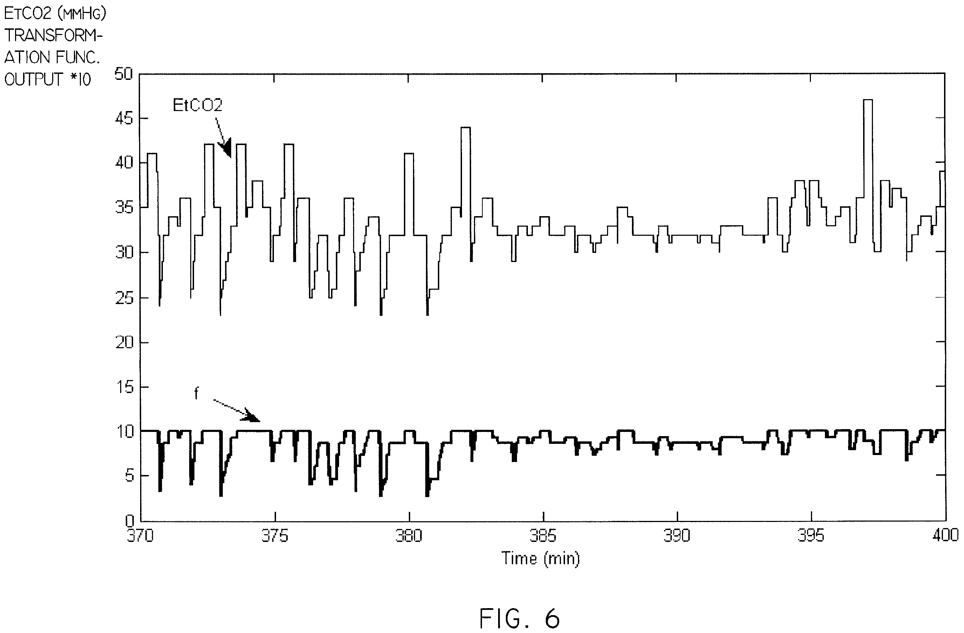

Fig. 5 shows a transformation function for $EtCO_2$ with the exemplary values of a=20, b=35, c=45 and d=70. Fig. 6 shows the values of the CISS (here only for $EtCO_2$) over time. The parameters may depend on the subject's age, medical history and/or the like.

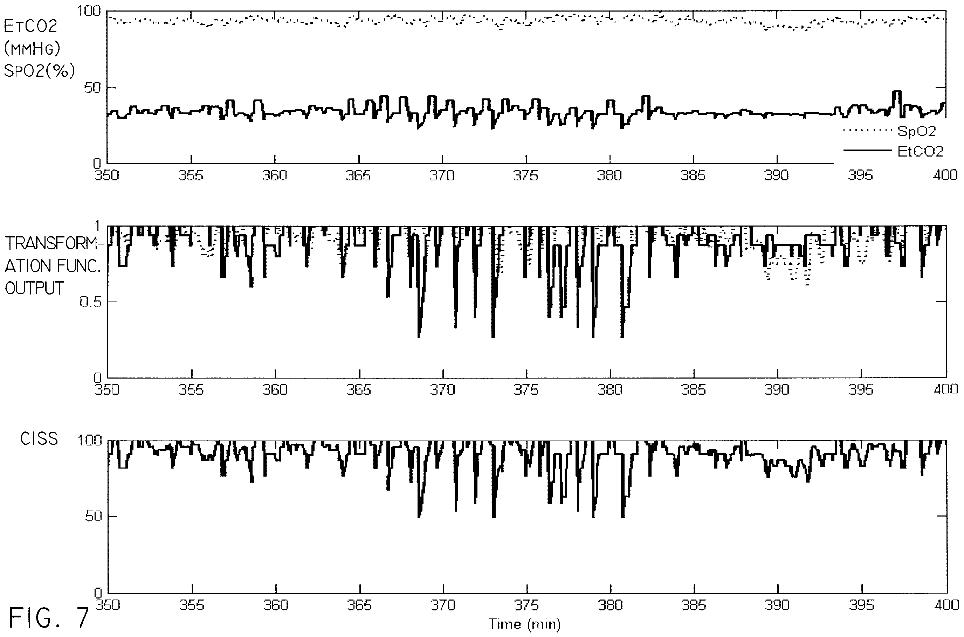

The use of linear combination of the two values, $EtCO_2$ and $SpO_2$, to generate CISS, is demonstrated in Fig. 7.

**Example 2**

An example of functions and generation of sleep scores for the event-based type of calculation are presented below.

An integrative function is applied over the range of the detected event. Its parameters are the parameter's value (x), the base-line (prior to the event) (BL), the length of the event (t), the differences between the minimum value and the base-line, the maximal differences and the minimum value. In the following are several examples for such function:

$$f = (max(BL - x))t^{0.5}$$

$$f = \frac{(BL - X)^2}{t}$$

$$f = \frac{(BL - X)^2 g(min(x))}{t}$$

$$g(x) = \begin{cases} 1, & x > 94 \\ 1.5, & 89 \le x \le 94 \\ 2, & x < 89 \end{cases}$$

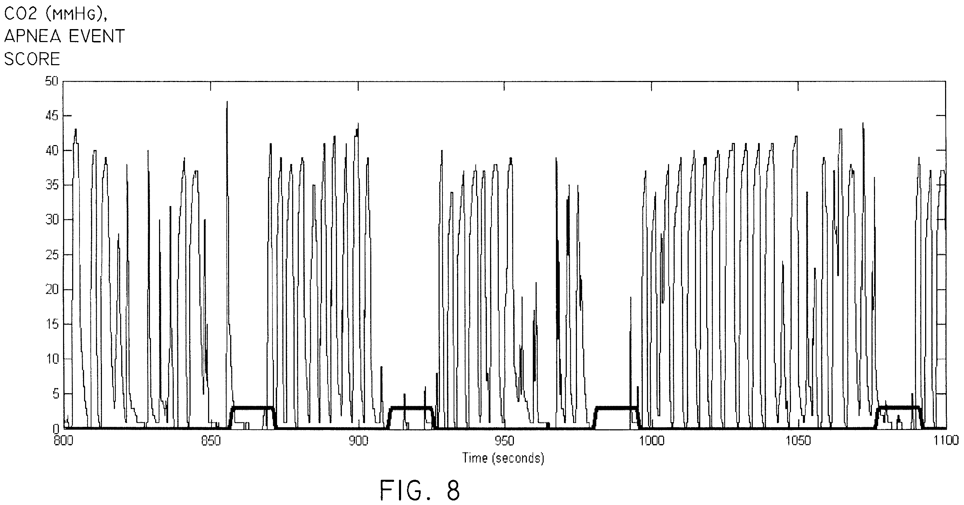

Fig. 8 shows $CO_2$ concentrations (waveforms) with continuous scoring between 0 and 10.

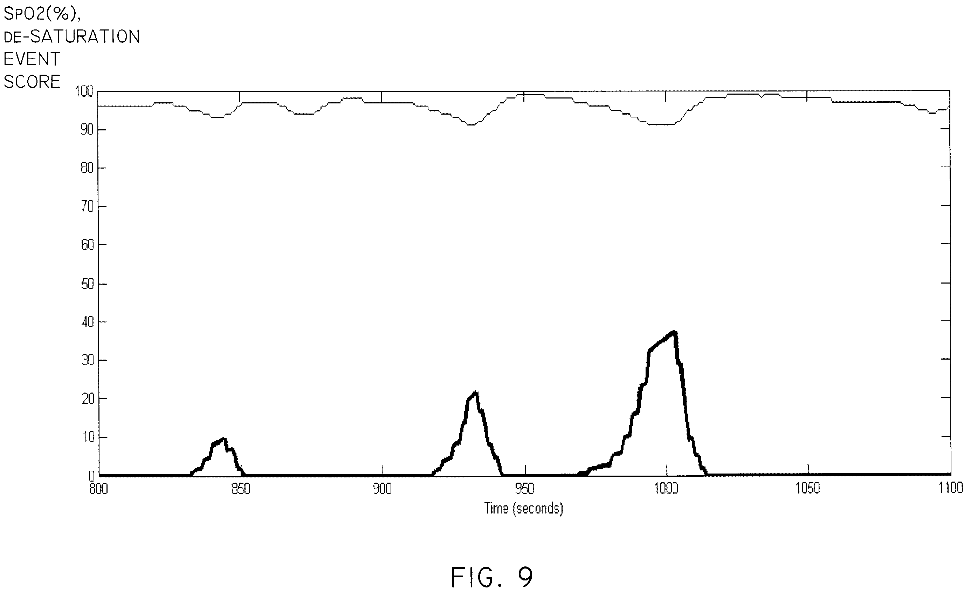

Fig. 9 shows $SpO_2$ values and the calculated score, 0 to 100.

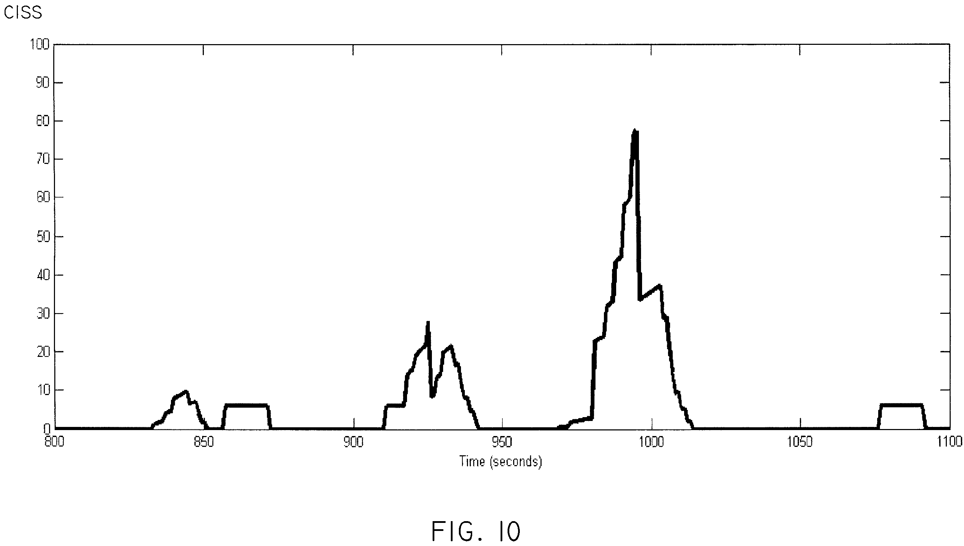

Fig. 10 shows the integrated score, 0 to 100.

In the description and claims of the application, each of the words "comprise" "include" and "have", and forms thereof, are not necessarily limited to members in a list with which the words may be associated.

**Claims**

1. A method for automatic sleep test analysis, the method comprising:

    continuously monitoring, using a pulse oximeter, oxygen saturation values of a patient;
    continuously monitoring, using a capnograph, exhaled carbon dioxide (CO2) values of the patient; and
    computing and outputting a continuous integrated sleep score based on a sequential analysis of the oxygen saturation values and the exhaled CO2 values, wherein the continuous integrated sleep score is indicative of the patient's breathing-related sleep quality during at least a portion of the sleep test;
    further comprising
    detecting multiple events indicative of ineffective breathing exhibited in one or more of the oxygen saturation values and the CO2 values; and
    computing a weighting function for the detected events, wherein each event is assigned a suitable weight;

        wherein the suitable weight for each event is based on applying an integrative function to the oxygen saturation values and CO2 values over a duration of each event,
        wherein the integrative function comprises the following parameters:

            the oxygen saturation values and CO2 values over the duration of the event;
            baseline oxygen saturation and CO2 values measured prior to the event; and the duration of the event.

2. The method according to claim 1, wherein the integrative function further comprises at least one parameter selected from the group consisting of:

    a difference between minimum oxygen saturation and CO2 values over the duration of the event and the baseline oxygen saturation and CO2 values;
a maximal difference between minimum oxygen saturation and CO2 values over the duration of the event and the baseline oxygen saturation and CO2 values; and
minimal oxygen saturation and CO2 values over the duration of the event.

3. The method according to claim 1, wherein the sequential analysis comprises applying to the oxygen saturation values and the exhaled CO2 values an analysis method selected from the group consisting of: a linear regression model, a non-linear regression model, fuzzy logic, a Bayesian network, a decision tree, a neural network, a radial base function and an expert system.

4. The method according to claim 1, further comprising computing, from the continuous integrated sleep score, an integrated sleep score for each of a plurality of time windows in the sleep test.

5. The method according to claim 4, wherein the time windows are moving time windows.

6. The method according to claim 5,
wherein the moving time windows are each of a static length; or
further comprising dynamically changing a length of at least some of the moving time windows.

7. The method according to claim 1,
further comprising:

monitoring one or more additional parameters selected from the group consisting of: heart rate, encephalogram (EEG), breath flow and chest movement,
wherein the computing of the continuous integrated sleep score is further based on one or more of the additional parameters;
or
further comprising summarizing at least a portion of the continuous integrated sleep score, to generate a summarized sleep quality score.

8. A system for automatic sleep test analysis, the system comprising:

a pulse oximeter for continuously monitoring oxygen saturation values of a patient;
a capnograph for continuously monitoring exhaled carbon dioxide (CO2) values of the patient; and
a computing unit configured to compute and output a continuous integrated sleep score based on a sequential analysis of the oxygen saturation values and the exhaled CO2 values, wherein the continuous integrated sleep score is indicative of the patient's breathing-related sleep quality during at least a portion of the sleep test;
wherein the computing unit is further configured to:

detect multiple events indicative of ineffective breathing exhibited in one or more of the oxygen saturation values and the CO2 values; and
compute a weighting function for the detected events, wherein each event is assigned a suitable weight;

wherein the suitable weight for each event is based on applying an integrative function to the oxygen saturation values and CO2 values over a duration of each event,
wherein the integrative function comprises the following parameters:

the oxygen saturation values and CO2 values over the duration of the event;
baseline oxygen saturation and CO2 values measured prior to the event; and the duration of the event.

9. The system according to claim 8, wherein the integrative function further comprises at least one parameter selected from the group consisting of:

a difference between minimum oxygen saturation and CO2 values over the duration of the event and the baseline oxygen saturation and CO2 values;
a maximal difference between minimum oxygen saturation and CO2 values over the duration of the event and the baseline oxygen saturation and CO2 values; and

minimal oxygen saturation and CO2 values over the duration of the event.

10. The system according to claim 8, wherein the sequential analysis comprises applying to the oxygen saturation values and the exhaled CO2 values an analysis method selected from the group consisting of: a linear regression model, a non-linear regression model, fuzzy logic, a Bayesian network, a decision tree, a neural network, a radial base function and an expert system.

11. The system according to claim 8, wherein the computing unit is further configured to compute, from the continuous integrated sleep score, an integrated sleep score for each of a plurality of time windows in the sleep test.

12. The system according to claim 11,
    wherein the time windows are moving time windows,
    preferably
    wherein the moving time windows are each of a static length; or
    wherein the computing unit is further configured to dynamically change a length of at least some of the moving time windows.

13. The system according to claim 8,
    further comprising at least one apparatus selected from the group consisting of:

    a heart rate monitor;
    an electroencephalogram (EEG) monitor;
    a breath flow sensor; and
    a chest movement sensor,

    wherein the computing is further based on data received by the computing unit from one or more of the apparatuses; or
    wherein the computing unit is further configured to summarize at least a portion of the continuous integrated sleep score, to generate a summarized sleep quality score.

**Patentansprüche**

1. Verfahren für eine automatische Schlaftestanalyse, wobei das Verfahren Folgendes umfasst:

   fortlaufendes Überwachen, unter Verwendung eines Pulsoximeters, von Sauerstoffsättigungswerten eines Patienten;
   fortlaufendes Überwachen, unter Verwendung eines Kapnographen, von Werten von ausgeatmetem Kohlendioxid (CO2) des Patienten; und
   Berechnen und Ausgeben einer fortlaufenden integrierten Schlafbewertung basierend auf einer sequenziellen Analyse der Sauerstoffsättigungswerte und der Werte von ausgeatmetem CO2, wobei die fortlaufende integrierte Schlafbewertung eine atmungsbedingte Schlafqualität des Patienten während wenigstens einem Anteil des Schlaftests anzeigt;
   das ferner Folgendes umfasst: Erfassen mehrerer Ereignisse, die eine ineffektive Atmung anzeigen, die sich den Sauerstoffsättigungswerten und/oder den CO2-Werten zeigt; und
   Berechnen einer Gewichtungsfunktion für die erfassten Ereignisse, wobei jedem Ereignis eine geeignete Gewichtung zugewiesen wird;
   wobei die geeignete Gewichtung für jedes Ereignis auf einem Anwenden einer integrativen Funktion auf die Sauerstoffsättigungswerte und die CO2-Werte über eine Dauer jedes Ereignisses basiert, wobei die integrative Funktion die folgenden Parameter umfasst:

       die Sauerstoffsättigungswerte und die CO2-Werte über die Dauer des Ereignisses;
       Basiswerte von Sauerstoffsättigung und CO2, die vor dem Ereignis gemessen werden; und
       die Dauer des Ereignisses.

2. Verfahren nach Anspruch 1, wobei die integrative Funktion ferner wenigstens einen Parameter umfasst, der aus der Gruppe ausgewählt ist, die aus Folgendem besteht:

   einem Unterschied zwischen minimalen Sauerstoffsättigungs- und CO2-Werten über die Dauer des Ereignisses und den Basiswerten von Sauerstoffsättigung und CO2;
einem maximalen Unterschied zwischen den minimalen Sauerstoffsättigungs- und CO2-Werten über die Dauer des Ereignisses und den Basiswerten von Sauerstoffsättigung und CO2; und
minimalen Sauerstoffsättigungs- und CO2-Werten über die Dauer des Ereignisses.

**3.** Verfahren nach Anspruch 1, wobei die sequenzielle Analyse das Anwenden auf die Sauerstoffsättigungswerte und die Werte von ausgeatmetem CO2 eines Analyseverfahrens umfasst, das aus der Gruppe ausgewählt ist, die aus Folgendem besteht:
einem linearen Regressionsmodell, einem nichtlinearen Regressionsmodell, Fuzzy-Logik, einem Bayes-Netz, einem Entscheidungsbaum, einem neuronalen Netz, einer radialen Basisfunktion und einem Expertensystem.

**4.** Verfahren nach Anspruch 1, das ferner das Berechnen, aus der fortlaufenden integrierten Schlafbewertung, einer integrierten Schlafbewertung für jedes von mehreren Zeitfenstern in dem Schlaftest umfasst.

**5.** Verfahren nach Anspruch 4, wobei die Zeitfenster bewegliche Zeitfenster sind.

**6.** Verfahren nach Anspruch 5, wobei die beweglichen Zeitfenster jeweils eine statische Länge besitzen; oder
das ferner ein dynamisches Ändern einer Länge wenigstens einiger der beweglichen Zeitfenster umfasst.

**7.** Verfahren nach Anspruch 1, das ferner Folgendes umfasst:
Überwachen eines oder mehrerer zusätzlicher Parameter, die aus der Gruppe ausgewählt sind, die aus Folgendem besteht:

Herzfrequenz, Enzephalogramm (EEG), Atemfluss und Brustbewegung, wobei das Berechnen der fortlaufenden integrierten Schlafbewertung ferner auf einem oder mehreren der zusätzlichen Parameter basiert; oder
das ferner ein Zusammenfassen wenigstens eines Anteils der fortlaufenden integrierten Schlafbewertung umfasst, um eine zusammengefasste Schlafqualitätsbewertung zu erzeugen.

**8.** System für die automatische Schlaftestanalyse, wobei das System Folgendes umfasst:

ein Pulsoximeter zum fortlaufenden Überwachen von Sauerstoffsättigungswerten eines Patienten;
einen Kapnographen zum fortlaufenden Überwachen der Werte von ausgeatmetem Kohlendioxid (CO2) des Patienten; und
eine Recheneinheit, die konfiguriert ist, um basierend auf einer sequenziellen Analyse der Sauerstoffsättigungswerte und der Werte von ausgeatmetem CO2 eine fortlaufende integrierte Schlafbewertung zu berechnen und auszugeben, wobei die fortlaufende integrierte Schlafbewertung die atembezogene Schlafqualität des Patienten während wenigstens eines Anteils des Schlaftests anzeigt;
wobei die Recheneinheit ferner für Folgendes konfiguriert ist:

Erfassen mehrerer Ereignisse, die eine ineffektive Atmung anzeigen, die sich in den Sauerstoffsättigungswerten und/oder den CO2-Werten zeigen; und
Berechnen einer Gewichtungsfunktion für die erfassten Ereignisse, wobei jedem Ereignis eine geeignete Gewichtung zugewiesen wird;

wobei die geeignete Gewichtung für jedes Ereignis auf dem Anwenden einer integrativen Funktion auf die Sauerstoffsättigungswerte und die CO2-Werte über eine Dauer jedes Ereignisses basiert, wobei die integrative Funktion die folgenden Parameter umfasst:

die Sauerstoffsättigungswerte und die CO2-Werte über die Dauer des Ereignisses;
Basiswerte von Sauerstoffsättigung und CO2, die vor dem Ereignis gemessen werden; und
die Dauer des Ereignisses.

**9.** System nach Anspruch 8, wobei die integrative Funktion ferner wenigstens einen Parameter umfasst, der aus der Gruppe ausgewählt ist, die aus Folgendem besteht:

einem Unterschied zwischen minimalen Sauerstoffsättigungs- und CO2-Werten über die Dauer des Ereignisses und den Basiswerten von Sauerstoffsättigung und CO2;
einem maximalen Unterschied zwischen den minimalen Sauerstoffsättigungs- und CO2-Werten über die Dauer des Ereignisses und den Basiswerten von Sauerstoffsättigung und CO2; und
minimalen Sauerstoffsättigungs- und CO2-Werten über die Dauer des Ereignisses.

10. System nach Anspruch 8, wobei die sequenzielle Analyse das Anwenden auf die Sauerstoffsättigungswerte und die Werte von ausgeatmetem CO2 eines Analyseverfahrens umfasst, das aus der Gruppe ausgewählt ist, die aus Folgendem besteht:
einem linearen Regressionsmodell, einem nichtlinearen Regressionsmodell, Fuzzy-Logik, einem Bayes-Netz, einem Entscheidungsbaum, einem neuronalen Netz, einer radialen Basisfunktion und einem Expertensystem.

11. System nach Anspruch 8, wobei die Recheneinheit ferner konfiguriert ist, um aus der fortlaufenden integrierten Schlafbewertung eine integrierte Schlafbewertung für jedes von mehreren Zeitfenstern in dem Schlaftest zu berechnen.

12. System nach Anspruch 11, wobei die Zeitfenster bewegliche Zeitfenster sind, vorzugsweise wobei die beweglichen Zeitfenster jeweils eine statische Länge besitzen; oder
wobei die Recheneinheit ferner konfiguriert ist, um eine Länge von wenigstens einigen der beweglichen Zeitfenster dynamisch zu ändern.

13. System nach Anspruch 8, das ferner wenigstens eine Vorrichtung umfasst, die aus der Gruppe ausgewählt ist, die aus Folgendem besteht:

einer Herzfrequenzüberwachungseinrichtung;
einer Elektroenzephalogramm(EEG)-Überwachungseinrichtung;
einem Atemflusssensor; und
einem Brustbewegungssensor,
wobei das Berechnen ferner auf Daten basiert, die durch die Recheneinheit von einer oder mehreren der Vorrichtungen empfangen werden; oder
wobei die Recheneinheit ferner konfiguriert ist, um wenigstens einen Anteil der fortlaufenden integrierten Schlafbewertung zusammenzufassen, um eine zusammengefasste Schlafqualitätsbewertung zu erzeugen.

**Revendications**

1. Procédé d'analyse de test de sommeil automatique, le procédé comprenant :

la surveillance continue, à l'aide d'un oxymètre de pouls, de valeurs de saturation en oxygène d'un patient ;
la surveillance continue, à l'aide d'un capnographe, de valeurs de dioxyde de carbone (CO2) expiré par le patient ; et
le calcul et la fourniture en sortie d'un score de sommeil intégré continu sur la base d'une analyse séquentielle des valeurs de saturation en oxygène et des valeurs de CO2 expiré, dans lequel le score de sommeil intégré continu indique la qualité de sommeil du patient associée à sa respiration pendant au moins une partie du test de sommeil ;
comprenant en outre la détection de multiples événements indiquant une respiration inefficace présentée dans une ou plusieurs des valeurs de saturation en oxygène et des valeurs de CO2 ; et
le calcul d'une fonction de pondération pour les événements détectés, dans lequel chaque événement se voit attribuer un poids approprié ;
dans lequel le poids approprié pour chaque événement est basé sur l'application d'une fonction intégrative aux valeurs de saturation en oxygène et aux valeurs de CO2 sur une durée de chaque événement, dans lequel la fonction intégrative comprend les paramètres suivants :

les valeurs de saturation en oxygène et les valeurs de CO2 sur la durée de l'événement ;
les valeurs de base de saturation en oxygène et de CO2 mesurées avant l'événement ; et
la durée de l'événement.

2. Procédé selon la revendication 1, dans lequel la fonction intégrative comprend en outre au moins un paramètre choisi dans le groupe constitué par :

une différence entre les valeurs minimales de saturation en oxygène et de CO2 sur la durée de l'événement et les valeurs de base de saturation en oxygène et de CO2 ;
une différence maximale entre les valeurs minimales de saturation en oxygène et de CO2 sur la durée de l'événement et les valeurs de base de saturation en oxygène et de CO2 ; et
des valeurs minimales de saturation en oxygène et de CO2 sur la durée de l'événement.

**3.** Procédé selon la revendication 1, dans lequel l'analyse séquentielle comprend l'application aux valeurs de saturation en oxygène et aux valeurs de CO2 expiré d'un procédé d'analyse choisi dans le groupe constitué par :
un modèle de régression linéaire, un modèle de régression non linéaire, une logique floue, un réseau bayésien, un arbre de décision, un réseau neuronal, une fonction de base radiale et un système expert.

**4.** Procédé selon la revendication 1, comprenant en outre le calcul, à partir du score de sommeil intégré continu, d'un score de sommeil intégré pour chaque fenêtre temporelle d'une pluralité de fenêtres temporelles dans le test de sommeil.

**5.** Procédé selon la revendication 4, dans lequel les fenêtres temporelles sont des fenêtres temporelles mobiles.

**6.** Procédé selon la revendication 5, dans lequel les fenêtres temporelles mobiles sont chacune d'une longueur statique ; ou
comprenant en outre le changement dynamique d'une longueur d'au moins certaines des fenêtres temporelles mobiles.

**7.** Procédé selon la revendication 1, comprenant en outre :
la surveillance d'un ou de plusieurs paramètres supplémentaires choisis dans le groupe constitué par :

la fréquence cardiaque, l'encéphalogramme (EEG), le débit respiratoire et les mouvements de la poitrine, dans lequel le calcul du score de sommeil intégré continu est en outre basé sur un ou plusieurs des paramètres supplémentaires ; ou
comprenant en outre le résumé d'au moins une partie du score de sommeil intégré continu de façon à générer un score résumé de qualité de sommeil.

**8.** Système d'analyse automatique du sommeil, le système comprenant :

un oxymètre de pouls permettant de surveiller en continu les valeurs de saturation en oxygène d'un patient ;
un capnographe permettant de surveiller en continu les valeurs de dioxyde de carbone (CO2) expiré par le patient ; et
une unité de calcul conçue pour calculer et fournir en sortie un score de sommeil intégré continu sur la base d'une analyse séquentielle des valeurs de saturation en oxygène et des valeurs de CO2 expiré, dans lequel le score de sommeil intégré continu indique la qualité de sommeil du patient associée à sa respiration pendant au moins une partie du test de sommeil ;
dans lequel l'unité de calcul est en outre conçue pour :

détecter plusieurs événements indiquant une respiration inefficace présentée dans une ou plusieurs des valeurs de saturation en oxygène et des valeurs de CO2 ; et
calculer une fonction de pondération pour les événements détectés, dans lequel chaque événement se voit attribuer un poids approprié ;

dans lequel le poids approprié pour chaque événement est basé sur l'application d'une fonction intégrative aux valeurs de saturation en oxygène et aux valeurs de CO2 sur une durée de chaque événement, dans lequel la fonction intégrative comprend les paramètres suivants :

les valeurs de saturation en oxygène et les valeurs de CO2 sur la durée de l'événement ;
les valeurs de base de saturation en oxygène et de CO2 mesurées avant l'événement ; et
la durée de l'événement.

**9.** Système selon la revendication 8, dans lequel la fonction intégrative comprend en outre au moins un paramètre choisi dans le groupe constitué par :

une différence entre les valeurs minimales de saturation en oxygène et de CO2 sur la durée de l'événement et les valeurs de base de saturation en oxygène et de $CO_2$ ;
une différence maximale entre les valeurs minimales de saturation en oxygène et de $CO_2$ sur la durée de l'événement et les valeurs de base de saturation en oxygène et de $CO_2$ ; et
des valeurs minimales de saturation en oxygène et de $CO_2$ sur la durée de l'événement.

**10.** Système selon la revendication 8, dans lequel l'analyse séquentielle comprend l'application aux valeurs de saturation en oxygène et aux valeurs de $CO_2$ expiré d'un procédé d'analyse choisi dans le groupe constitué par :
un modèle de régression linéaire, un modèle de régression non linéaire, une logique floue, un réseau bayésien, un arbre de décision, un réseau neuronal, une fonction de base radiale et un système expert.

**11.** Système selon la revendication 8, dans lequel l'unité de calcul est en outre conçue pour calculer, à partir du score de sommeil intégré continu, un score de sommeil intégré pour chaque fenêtre temporelle d'une pluralité de fenêtres temporelles dans le test de sommeil.

**12.** Système selon la revendication 11, dans lequel les fenêtres temporelles sont des fenêtres temporelles mobiles, dans lequel les fenêtres temporelles mobiles sont de préférence chacune d'une longueur statique ; ou
dans lequel l'unité de calcul est en outre conçue pour changer dynamiquement une longueur d'au moins certaines des fenêtres temporelles mobiles.

**13.** Système selon la revendication 8, comprenant en outre au moins un appareil choisi dans le groupe constitué par
un moniteur de fréquence cardiaque ;
un moniteur d'électroencéphalogramme (EEG) ;
un capteur de débit respiratoire ; et
un capteur de mouvement de la poitrine, dans lequel le calcul est en outre basé sur des données reçues par l'unité de calcul à partir d'un ou de plusieurs des appareils ; ou
dans lequel l'unité de calcul est en outre conçue pour résumer au moins une partie du score de sommeil intégré continu de façon à générer un score résumé de qualité de sommeil.

FIG. I

EP 2 584 966 B1

FIG. 2

MONITOR PARAMETER INPUT | WINDOW ANALYZED AT TIME Tn. | TRANSFORMATION FUNCTION TO BREATHING EFFECTIVENESS | SINGLE, INTEGRATED SCORE PER PARAMETER

FIG. 3

EP 2 584 966 B1

SINGLE INTEGRATED SCORE FOR WINDOW AT TIME T$_N$.

CONTINUOUS INTEGRATED SLEEP SCORE (CISS)

SLEEP QUALITY SCORE (SQS) FOR SLEEP PERIOD OR SUB PERIOD

AVERAGE OR STATISTICAL SUMMARY

0-10, 0-100 MATRIX (X,Y) OR GRAPHIC REPRESENTATION

(MINUTES)

FIG. 4

EP 2 584 966 B1

FIG. 5

FIG. 6

FIG. 7

CO2 (MMHG),
APNEA EVENT
SCORE

FIG. 8

EP 2 584 966 B1

SPO2(%),
DE-SATURATION
EVENT
SCORE

FIG. 9

FIG. 10

EP 2 584 966 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009063446 A2
- WO 2008029399 A2
- US 2007191697 A1